# FASCICULE DE BREVET EUROPEEN

(11) **EP 2 653 140 B1**
(45) Date de publication et mention de la délivrance du brevet: **04.03.2015**
(21) Numéro de dépôt: 13164262.1
(22) Date de dépôt: 18.04.2013
(51) Int. Cl.: A61F 13/02, A61L 15/18, A61F 13/84

(54) **Sparadrap et son utilisation**
Heftpflaster und seine Verwendung
Adhesive bandage and use thereof

(30) Priorité: 20.04.2012 FR 1253638
(43) Date de publication de la demande: 23.10.2013
(73) Titulaire: Henkoscience, 77410 Gressy (FR)
(72) Inventeur: Fontas, Valérie, 77410 GRESSY EN FRANCE (FR)
(74) Mandataire: Novagraaf Technologies

(56) Documents cités:
- EP-A1- 0 116 240
- EP-A1- 0 676 183
- WO-A1-2011/004126
- FR-A5- 2 096 036
- US-A- 3 935 363

## Description

Est ici concerné un sparadrap comprenant:
- une bande souple ayant au moins une face collante, pour coller, notamment à un tissu humain, tel la peau, et
- une partie de pansement fixée à ladite bande collante sur une partie seulement de la surface de ladite face collante.

Habituellement, les sparadraps sont inertes. Typiquement, la partie de pansement est une sorte de gaze.

Un problème que vise à résoudre le sparadrap précité est de pouvoir lier à une partie de la face collante une poudre ou granulat, telle une argile.

Une solution proposée consiste en ce que la partie de pansement comprenne un support poreux, non-tissé, maillé, à fibres naturelles ou synthétiques auquel est agglomérée une charge poudreuse, telle une argile, et présentant une première face davantage ainsi chargée que la seconde face opposée par laquelle le support est collé à la bande.

Ainsi, on va utiliser une interface comprenant le support non-tissé maillé dont la deuxième face sera suffisamment peu chargée en poudre pour pouvoir coller à la bande souple collante de support, par ses fibres.

A l'opposé, la première face chargée permettra d'utiliser cette charge, par exemple pour adoucir le contact avec la peau, puisqu'il s'agit d'une poudre. Traiter et/ou cicatriser peut aussi être visé, notamment avec de l'argile humidifiée.

Mouillée, une poudre agglomérée peut avoir un effet de traitement en couche mince. S'il s'agit d'une argile, elle devient alors une substance active dont les (ou du moins certains) effets sont connus.

On appellera ci-après :
- poudre ou poudreux(se) une substance sèche, qui peut couler, et présente une granulométrie inférieure à 10 microns.
- pansement, la partie du sparadrap qui ne colle pas au tissu concerné, tel la peau.

L'éventuel effet thérapeutique est indifférent.

Dans un état de stockage du sparadrap (1), la face collante est recouverte d'une pellicule pelable de protection, à retirer pour coller le sparadrap, et qui ne recouvre de préférence pas la partie de pansement.

Un autre problème peut être lié au retrait inopiné, non souhaité, de la poudre chargeant le support, notamment alors que la charge est sèche.

Une solution proposée est que, dans un état de stockage, le sparadrap soit conditionné dans une enveloppe fermée déchirable à la main, souple et lisse.

Ainsi, on limitera les contacts de la poudre avec des surfaces qui peuvent être assez rugueuses. On diminuera les pertes possibles par frottement. Par ailleurs, on évitera le contact prématuré avec l'humidité, y compris ambiante.

En particulier, s'il s'agit d'argile, un autre problème concerne l'incompatibilité entre le caractère typiquement non résistant à l'eau des sparadraps collants et l'activation par l'humidité (tel l'eau) d'une argile pour activer ses principes actifs.

Une solution ici proposée pour surmonter cette incompatibilité de principe est que la face collante (ou adhésive) comprenne une colle, telle une colle acrylique, qui demeure collante, même mouillée.

Ainsi, au lieu de rendre étanche à l'eau les sparadraps comme le sont certains (dits « waterproof »-étanche), on pourra mouiller la partie de pansement sans que celle-ci se détache (se décolle) ni que les autres zones collantes fassent de même.

Comme on le sait, les sparadraps « étanches » présentent typiquement le pansement au centre, sur une face, et une zone collante qui en fait tout le tour, continûment. La surface collante périphérique isole alors le pansement central de l'eau, la bande collante ne se laissant structurellement pas traverser par l'eau.

De façon différente, il est ici proposé, pour valoriser au mieux la partie de pansement, que la bande s'étende dans un plan suivant lequel elle présente une longueur, une largeur et deux bords opposés suivant cette longueur, la partie de pansement s'étendant sur toute la largeur de la bande.

Sur un autre aspect, il est par ailleurs conseillé que la première face soit saturée d'argile, laquelle est :
- sèche dans un état de stockage du sparadrap où il est alors stocké dans une boîte de conditionnement,
- et humide dans un état d'application du sparadrap sur la peau à laquelle on le colle alors.

A noter aussi que, dans le cadre de l'utilisation du sparadrap, on recommande, avant de le coller sur une peau, de mouiller préalablement (typiquement à l'eau) au moins la partie de pansement.

D'autres caractéristiques liées au présent sujet pourront encore apparaître dans la description du mode préféré de réalisation qui suit, présenté à titre non limitatif, en référence aux dessins annexés où :
- les figures 1, 2 et 3 montrent respectivement le sparadrap seul, avec ses pellicules pelables de protection, puis sans elles, alors prêt à coller, puis conditionné dans un emballage individuel,
- la figure 4 montre une boîte de stockage de tels sparadraps, secs,
- la figure 5 montre un sparadrap collé à la peau,
- et la figure 6 montre dans le sens de l'épaisseur la partie de pansement comprenant le support fibreux auquel est agglomérée une charge poudreuse.

Sur les figures, on voit donc un sparadrap 1 formant globalement un adhésif autocollant apte à adhérer à la peau 3 d'une personne. Il est alors physiocompatible.

Le sparadrap 1 comprend :
- une bande souple 5 qui a, dans l'exemple, une face collante ou adhésive 5a, pour coller, ici au tissu humain 3, et
- une partie de pansement 7.

La partie de pansement 7 est fixée à la bande collante 5a sur une partie seulement de la surface de ladite face collante, comme illustré. Elle est non-collante.

Cette partie de pansement 7 comprend un support 9 poreux, non-tissé, maillé, à fibres naturelles ou synthétiques auquel est agglomérée une charge poudreuse 11, telle une argile.

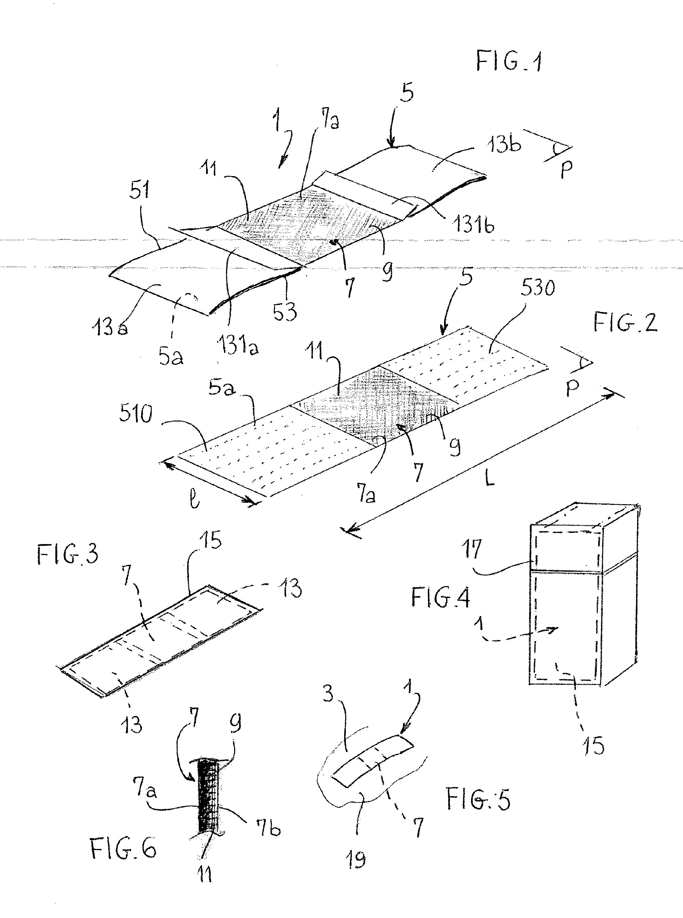

Comme montré plus clairement figure 6, une première face 7a de la partie de pansement 7 est davantage ainsi chargée que la seconde face opposée 7b de cette partie, par laquelle le support 9 est collé à la bande 5.

Pour favoriser le fait que la charge 11 ne se sépare pas significativement du support 9, qu'il soit sec ou mouillé (y compris donc lorsque le tout est immergé dans un liquide mouillage, tel de l'eau, typiquement à température ambiante), on recommande ce qui suit (voir pour toute information plus détaillé WO-A-2011004126) : On réalise le support souple 9 imprégné (au moins) d'argile 30a (à coeur et en surface) en pulvérisant sur un support ou substrat vierge souple, à structure aérée, un produit pâteux contenant de l'argile en mélange avec un liquide (typiquement de l'eau). Pour réaliser ce produit pâteux, l'argile est apportée sèche sous forme de particules. Pour imprégner à coeur le support 9 vierge, le produit pâteux pulvérisé est de préférence une pâte contenant, en masse de mélange, entre 30 et 60% d'argile et entre 40 et 70% de liquide, en particulier eau, voire alcool, ou combinaison. L'argile 30a utilisée sera favorablement phylitteuse et non gonflante. Un résultat particulièrement performant a été constaté avec une argile ayant une granulométrie entre 10 microns et 100 microns, et de préférence entre 20 microns et 30 microns. On recommande que l'argile à pulvériser soit plutôt de l'illite. Des additifs de renforcement des propriétés de l'argile peuvent être adjoints à ce mélange pâteux, tels extraits végétaux, arômes, huiles essentielles, molécules actives. L'argile demeurera largement majoritaire ; plus de 90%. Pour favoriser la projection, on conseille que le produit pâteux 3 contienne un taux de charge massique en argile inférieur à 42%.

En particulier, avec une charge d'argile, et pour alors limiter les pertes inopinées d'argile malgré sa (très) bonne adhésion à son support 9, on recommande que, dans un état de stockage du sparadrap, celui-ci soit conditionné dans une enveloppe 15 fermée, déchirable à la main, souple et lisse. De cette façon, on limitera les frottements et contacts rugueux avant utilisation.

Encore dans le cas d'une charge d'argile, on conseille par ailleurs que la première face 7a de la partie de pansement 7 soit saturée d'argile, laquelle est donc sèche dans l'état de stockage de sparadrap, où il est alors stocké dans une boîte de conditionnement 17 (figure 4), tandis que ce sparadrap est humide dans un état d'application sur la peau 19 à laquelle on le colle alors (figure 5).

La boîte 17 sera de préférence une boîte rigide renfermant une série de sparadraps (conditionnés donc secs) et de préférence chacun disposés dans une enveloppe 15 fermée.

En particulier figures 1,2, on voit par ailleurs que la bande souple 5 s'étend dans un plan P suivant lequel elle présente une longueur L, une largeur 1 et deux bords opposés 51,53, suivant cette longueur.

Egalement suivant la longueur L, la partie de pansement 7 s'étend sensiblement au centre, entre des zones collantes 510,530 de la face 5a.

De préférence :
- les zones collantes 510,530 sont d'égales surfaces,
- et/ou leur surface est un peu (10 à 30%) inférieure à celle de la partie de pansement 7.

Egalement de préférence, la partie de pansement 7 s'étend sur toute la largeur 1 de la bande 5. Ainsi il ne s'agit pas d'un pansement/sparadrap étanche à l'eau (dit « water-proof »).

Notamment figure 1, on voit que, dans un état de stockage du sparadrap, la face collante 5a est recouverte d'(au moins) une pellicule pelables de protection 13, à retirer pour coller le sparadrap, et qui ne recouvre pas la partie de pansement. Ainsi on pourra notamment mouiller aisément le pansement, sans altérer la zone collante protégée par la pellicule 13.

Comme illustré, on conseille de prévoir deux pellicules pelables de protection 13a,13b appliquées (collées) respectivement sur les zones collantes 510,530.

De préférence, la/chaque pellicule de protection 13,13a,13b, qui est souple, est pourvue d'un rabat 131a,131b situé en limite de la partie de pansement 7, pour faciliter le retrait de la pellicule concernée. On aura compris que les rabats 131a,131b ne sont pas collés aux faces collantes.

Comme on l'a mentionné précédemment, l'utilisation d'une charge d'argile sur la partie de pansement 7 implique un intérêt, au moins dans certains cas, à humidifier le pansement pour favoriser l'impact de l'argile sur la peau à laquelle on colle alors le sparadrap.

Pour encore favoriser cette manière d'utiliser le sparadrap, on recommande que la face collante 5a comprenne une colle, telle une colle acrylique, qui demeure collante, même mouillée.

Ainsi, en particulier avec une telle précaution, un aspect de la solution proposée consiste en ce qu'on utilise le sparadrap 1 en le collant sur la peau d'une personne, en en ayant préalablement mouillée au moins la partie de pansement 7.

## Revendications

1. Sparadrap (1) comprenant :
- une bande souple (5) ayant au moins une face collante (5a), pour coller, notamment à un tissu humain, tel la peau, et
- une partie de pansement (7) fixée à ladite bande collante sur une partie seulement de la surface de ladite face collante,
la partie de pansement (7) comprenant un support (9) poreux et non-tissé, à fibres naturelles ou synthétiques auquel est agglomérée une charge poudreuse (11), telle une argile, et présentant une première face (7a) davantage chargée que la seconde face opposée (7b) par laquelle ledit support (9) est collé à la bande (5), **caractérisé en ce que**, dans un état de stockage du sparadrap (1), la face collante (5a) est recouverte d'une pellicule pelable de protection, à retirer pour coller le sparadrap (1), et qui ne recouvre pas la partie de pansement (7).

2. Sparadrap selon la revendication 1, **caractérisé en ce que**, dans un état de stockage du sparadrap (1), il est conditionné dans une enveloppe fermée déchirable à la main, souple et lisse.

3. Sparadrap selon l'une des revendication 1 ou 2, **caractérisé en ce que** la face collante (5a) comprend une colle, telle une colle acrylique, qui demeure collante, même mouillée.

4. Sparadrap selon l'une des revendications 1 à 3, **caractérisé en ce que** la bande (5) s'étend dans un plan suivant lequel elle présente une longueur, une largeur et deux bords opposés suivant cette longueur, la partie de pansement (7) s'étendant sur toute la largeur de la bande (5).

5. Sparadrap selon l'une des revendications 2 à 4, **caractérisé en ce que** la première face (7a) est saturée d'argile (30a), laquelle est sèche dans un état de stockage du sparadrap (1), où il est alors stocké dans une boîte de conditionnement, et humide dans un état à coller du sparadrap (1) où la protection n'a pas encore été retirée.

## Patentansprüche

1. Heftpflaster (1), umfassend:
- einen biegsamen Streifen (5), der mindestens eine Klebefläche (5a) aufweist, um insbesondere auf einem menschlichen Gewebe, wie der Haut, zu kleben, und
- einen Verbandteil (7), der an dem Klebestreifen auf nur einem Teil der Oberfläche der Klebefläche befestigt ist,
wobei der Verbandteil (7) einen porösen Träger (9) aus Faservlies mit natürlichen oder synthetischen Fasern umfasst, auf dem ein pulveriger Stoff (11), wie Tonerde, durch Agglomeration aufgebracht ist, und der eine erste Seite (7a) aufweist, die stärker beaufschlagt ist als die zweite gegenüberliegende Seite (7b), mit der der Träger (9) auf den Streifen (5) geklebt wird,
**dadurch gekennzeichnet, dass** in einem Aufbewahrungszustand des Heftpflasters (1) die Klebefläche (5a) mit einer abziehbaren Schutzfolie bedeckt ist, die zum Aufkleben des Heftpflasters (1) abzuziehen ist und die den Verbandteil (7) nicht bedeckt.

2. Heftpflaster nach Anspruch 1, **dadurch gekennzeichnet, dass** in einem Aufbewahrungszustand des Heftpflasters (1) dieses in einer von Hand aufreißbaren, biegsamen und glatten geschlossenen Hülle verpackt ist.

3. Heftpflaster nach einem der Ansprüche 1 oder 2, **dadurch gekennzeichnet, dass** die Klebefläche (5a) einen Kleber, wie einen Acrylkleber, umfasst, der, auch wenn er feucht wird, klebend bleibt.

4. Heftpflaster nach einem der Ansprüche 1 bis 3, **dadurch gekennzeichnet, dass** sich der Streifen (5) in einer Ebene erstreckt, in der er eine Länge, eine Breite und zwei gegenüberliegende Ränder entlang dieser Länge aufweist, wobei sich der Verbandteil (7) über die gesamte Breite des Streifens (5) erstreckt.

5. Heftpflaster nach einem der Ansprüche 2 bis 4, **dadurch gekennzeichnet, dass** die erste Seite (7a) mit Tonerde (30a) gesättigt ist, die in einem Aufbewahrungszustand des Heftpflasters (1), in dem es nun in einer Verpackungsschachtel aufbewahrt wird, trocken ist, und die in einem Zustand zum Aufkleben des Heftpflasters (1), in dem der Schutz noch nicht abgezogen wurde, feucht ist.

## Claims

1. Sticking plaster (1) including:
- a flexible strip (5) having at least one adhesive face (5a) for sticking it, notably to a human tissue, such as skin, and
- a dressing portion (7) fixed to said adhesive strip over a portion only of the surface of said adhesive face,
the dressing portion (7) including a porous nonwoven support (9) made of natural or synthetic fibres combined with a powder filler (11), such as a clay filler, and having a first face (7a) more loaded than the opposite second face (7b) by which said support (9) is stuck to the strip (5),
**characterized in that**, in a storage state of the sticking plaster (1), the adhesive face (5a) is covered with a peelable protective film, to be removed to stick on the sticking plaster (1), that does not cover the dressing portion (7).

2. Sticking plaster according to claim 1, **characterized in that**, in a storage state of the sticking plaster (1), it is packaged in a sealed envelope that is tearable by hand, flexible and smooth.

3. Sticking plaster according to either one of claims 1 or 2, **characterized in that** the adhesive face (5a) includes an adhesive, such as an acrylic adhesive, which remains sticky even when wet.

4. Sticking plaster according to any one of claims 1 to 3, **characterized in that** the strip (5) extends in a plane in which it has a length, a width and two opposite edges along this length, the dressing portion (7) extending over the entire width of the strip (5).

5. Sticking plaster according to any one of claims 2 to 4, **characterized in that** the first face (7a) is saturated with clay (30a), which is dry in a storage state of the sticking plaster (1), in which it is stored in a packaging box, and wet in a state for sticking on the sticking plaster (1) when the protection has not yet been removed.
